(19) Europäisches Patentamt
European Patent Office
Office européen des brevets

(11) **EP 3 458 816 B1**

(12) **EUROPEAN PATENT SPECIFICATION**

(45) Date of publication and mention
of the grant of the patent:
**28.07.2021 Bulletin 2021/30**

(21) Application number: **17799768.1**

(22) Date of filing: **11.05.2017**

(51) Int Cl.:
*G01F 3/26* *(2006.01)*          *C02F 11/04* *(2006.01)*
*C10L 3/10* *(2006.01)*          *C12M 1/107* *(2006.01)*
*C12M 1/34* *(2006.01)*          *G01F 3/30* *(2006.01)*
*G01F 1/74* *(2006.01)*          *C12C 11/00* *(2006.01)*

(86) International application number:
**PCT/SE2017/050477**

(87) International publication number:
**WO 2017/200464 (23.11.2017 Gazette 2017/47)**

(54) **GAS MEASUREMENT METHOD AND DEVICE FOR BATCH FERMENTATION AND IN-VITRO ANALYSIS PLATFORMS**

GASMESSVERFAHREN UND MESSGERÄT FÜR CHARGENWEISE FERMENTATION UND IN-VITRO-ANALYSEPLATTFORMEN

PROCÉDÉ ET DISPOSITIF DE MESURE DE GAZ POUR FERMENTATION DISCONTINUE ET PLATEFORMES D'ANALYSE IN VITRO

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2016 SE 1650687**

(43) Date of publication of application:
**27.03.2019 Bulletin 2019/13**

(73) Proprietor: **BPC Instruments AB**
**223 63 Lund (SE)**

(72) Inventor: **LIU, Jing**
**226 48 Lund (SE)**

(74) Representative: **AWA Sweden AB**
**P.O. Box 5117**
**200 71 Malmö (SE)**

(56) References cited:
WO-A1-2010/120229     DE-A1- 4 006 508
GB-A- 2 530 571           US-A- 4 064 750
US-A- 4 064 750           US-A1- 2012 064 616

## Description

Field of the invention

The present invention relates to a method and device for gas analysis measurement intended to be utilized in batch fermentation assays including anaerobic and aerobic respiration analysis and/or *in-vitro* analysis with various applications in different industrial fields.

Technical Background

There are many well-known and used gas measurement technologies in the field and on the market today, including in the biogas production field and/or fermentation field in general. Furthermore, also gas measurements in the form of manometric methods are used in the field of animal nutrition.

The present invention is directed to a gas measurement method optimized for a batch fermentation assays and/or an *in-vitro* analysis, optimized in terms of measuring in a very accurate and precise way, e.g. for *in-vitro* analysis in the fields of animal or human nutrition, or gut microbiota evaluation.

Summary of the invention

The stated purpose above is achieved by a method for performing a gas analysis measurement, said method comprising directing a gas flow or gas sample to a gas flow measurement device, said gas flow measurement device comprising a gas inlet, a gas outlet, and a gas compartment with a definite and predefined inner geometric physical volume, the gas compartment having one gas accumulating end and one lifting end and having a pivoting element enabling the gas compartment to pivot upwards to release the gas contained therein and then downwards again to its initial position and operating according to the leverage effect, wherein the gas compartment is arranged in a closed wet space compartment, implying that the gas compartment is only connected to the outside via the gas inlet and the gas outlet, the gas compartment also having at least one sensor, for a volumetric measurement of the gas flow, wherein said method is performed for evaluating a batch fermentation and/or in an in-vitro analysis platform, and wherein using the gas flow measurement device is performed for evaluating the specific characteristics of animal feed/nutrition(s) or human food/nutrition(s) by determining the digestibility, or evaluating the activity of gut microbiota or yeast or bacteria by determining the fermentative capacity/production or bacterial metabolic activities.

According to a first aspect, the present invention is directed to a method according to appended claim 1, for performing a gas analysis measurement, said method comprising directing a gas flow or gas sample to a gas flow measurement device, said gas flow measuring device comprising a gas inlet and a gas compartment with a definite and predefined inner geometric physical volume and active volume, the gas compartment having one gas accumulating end and one lifting end and having a pivoting element enabling the gas compartment to pivot upwards to release the gas contained therein and then downwards again to its initial position and operating according to the leverage effect, wherein the gas compartment is arranged in a closed wet space compartment, implying that the gas compartment is only connected to the outside via a gas inlet and a gas outlet, the gas compartment also having at least one sensor, for a volumetric measurement of the gas flow, wherein the method is performed for evaluating a batch fermentation and/or in an in-vitro analysis platform.

As may be understood from above, the gas compartment is a flow cell which is arranged in a closed compartment, the closed compartment being a closed wet space. This arrangement, with a single flow cell arranged in a single closed wet space compartment, according to the present invention has several advantages. First of all, this arrangement according to the present invention allows a gas stream to be measured in more than one gas measuring device after removing a certain gas component, i.e. when several measuring devices according to the present invention are connected in series. Moreover, this implies that the present invention enables measuring both total gas and individual gas component types via analysis with the volumetric measuring method according to the present invention, i.e. without the need of direct gas composition analysis using expensive instruments like GC or infrared sensors. In this context it may be mentioned that known gas measuring devices, such as the ones disclosed in US 2012064616, WO2010120229, WO2010120230, US4064750 and GB2531331, do not have this type of arrangement as according to the present invention, i.e. with a single gas compartment or flow cell being arranged in a single closed wet space compartment. Thus, these devices cannot provide a solution for total gas measurement and individual gas component analysis without utilizing extra equipment, such as a GC or infrared sensors.

As may be understood from above, one very important aspect of the present invention is the step of a volumetric measurement of the gas flow and the link to the industrial applications of batch fermentation assay including anaerobic and aerobic respiration analysis and/or *in-vitro* analysis. This renders a very high accuracy with minimized risk of experimental variations and errors, as is the case of methods. Today only these manometric methods are used where the creation of an either overpressure or underpressure is the actual driver for the measurement. This is not true for the method according to the present invention where the gas measurement is volumetric and no over-pressure is needed.

Moreover, in relation to above it should be mentioned that the measurement may be performed continuously with real time data acquisition.

Furthermore, the method according to the present invention may be employed for several industrial applications. In general, the present invention may be employed for applications including biogas measurement analysis, animal feed analysis, gut microbiota evaluation, human nutrition analysis, greenhouse gas emission analysis, biochemical oxygen demand (BOD) analysis for water and wastewater, anammox (anaerobic ammonium oxidation) process for nitrogen removal in wastewater, bioethanol production, yeast fermentation, composability analysis, etc. The present invention is especially directed to applications involving analysis within the field of human nutrition, animal feed, BOD analysis, composability and greenhouse gas emission.

Brief description of the drawings

Fig. 1 shows one embodiment of gas flow measuring device suitable for a system according to the present invention.
Fig. 2a and b also show the gas flow measuring device according to fig. 1, however in this case all parts are totally separated. Fig. 2c shows yet another embodiment of the present invention, in this case where sealing is enabled by direct welding instead of using a sealing ring.
Fig. 3 shows a schematic block diagram of one possible method according to the present invention, where the steps thereof are presented in the blocks.
Fig. 4-6 show diagrams of different trials when the method according to the present invention was evaluated.

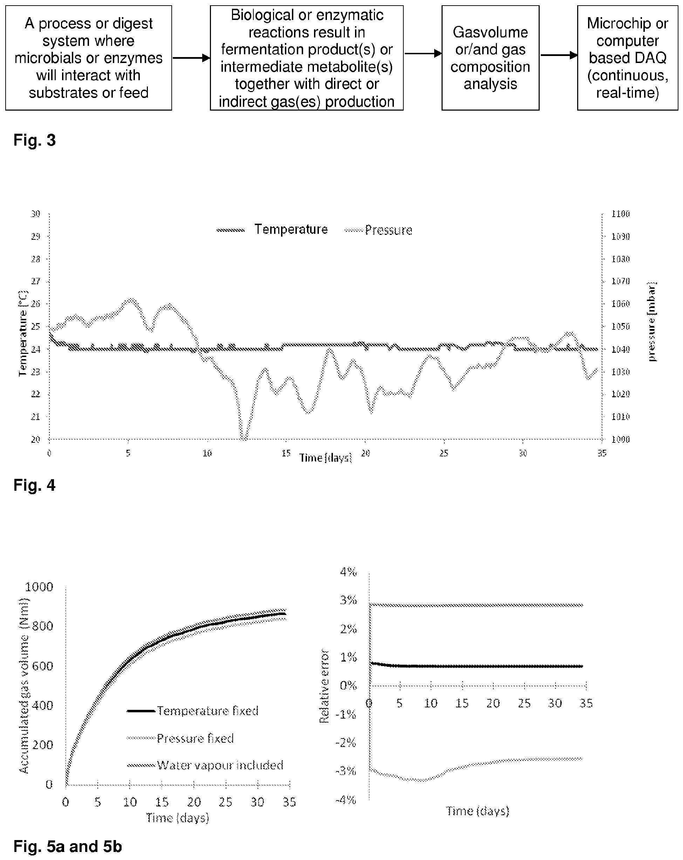

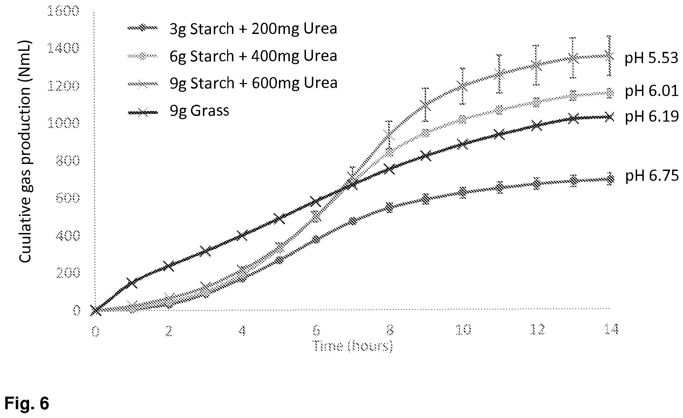

In these figures, fig. 4 shows a dynamic profile of temperature and pressure during the biochemical methane potential test, fig. 5 shows recorded accumulated volume of a biochemical methane potential test when different factors have been assumed constant, and the resulting error percentage over time for each situation, and fig. 6 shows accumulated gas volume in normalised mL over time, with final pH value.

Specific embodiments of the invention

Below specific embodiments of the present invention are discussed. First of all, and as hinted above, the method according to the present invention finds use in many different industrial applications. According to one specific embodiment, the volumetric measurement of the gas flow by using the gas flow measurement device is performed in an *in-vitro* analysis test. An *in-vitro* analysis according to the present invention may be employed for many different applications, e.g. in analysis of animal feeds, gut microbiota evaluation or nutrition and analysis of human food/nutrition, e.g. functional foods, such as dietary for special groups of people including patients, infants, old people, as well as individual dietary treatment used in sports et al. Therefore, according to the present invention, the method comprises that using the gas flow measurement device is performed for evaluating the specific characteristics of animal feed/nutrition(s) or human food/nutrition(s) by determining the digestibility, or evaluating probiotics and/or impact of prebiotics utilization to gut microbiota, or evaluating the metabolic activity of yeast or bacteria by determining the fermentative capacity/production or bacterial metabolic activities, or e.g. metabolic intermediates via direct or indirect gas emission.

With reference to animal nutrition, the specific characteristics of a specific animal feed may be determined by how well the feed is digested and related digestibility kinetics. There is a close association between rumen fermentation and gas production. This is also true for monogastric animals other than ruminants, e.g. pigs and poultry. Gas production measurement methods have been reported and used to determine digestibility of feeds, said methods being focused on pressure-based manometric measurements. This is a clear difference in relation to the present invention, which is a volumetric measurement.

The method according to the present invention has several other entry points into the field of animal nutrition, such as optimizing feeds in the animal feed sector including to screen feed samples or additives (e.g. enzymes, antibiotics, dicarboxylic acids, etc.) before moving on *in-vivo* tests.

With reference to human nutrition, the following may be mentioned. The gut microbiota is a highly specialized organ which directly impacts human health and disease. The digested food has an effect on the metabolic activities and species composition of the human colonic microbiota. An *in-vitro* technology, such as provided according to the present invention, presents a very interesting alternative to the today used *in-vivo* techniques either based on labor intensive chemical or microbial analysis or expensive analytical methods such as HPLC. A gas measurement in an *in-vitro* platform according to the present invention may be used for the nutritive evaluation of foods (diets, food advice, producers of food additives/supplements, infant nutrition, clinical nutrition, etc.) but may also be employed in the medical, healthcare and clinical analysis field. Alterations in the gut microbiota may be associated with specific dietary patterns and diseases. The gas species produced by the microbiota affect the status of the gut, and thereby the health status of a human object. This gas species may be used as biomarkers to assess gastrointestinal functions and the treatments of diseases related to the gastrointestinal tract.

With reference to the above disclosed, according to one specific embodiment of the present invention, the volumetric measurement of the gas flow or gas sample is performed as an *in-vitro* analysis test of the digestibility of the animal feed/nutrition(s) or the human food/nutrition(s).

There are many protocols available on how to perform *in-vitro* digestibility tests, and some of them utilize gas measurement. Corrections to standard conditions for temperature and pressure are often poorly described and/or presented using different standard values which could lead to differences of up to 10% in the corrected volume. Moreover, another factor that is not addressed in many corrections is the water content of a gas. All of these problems are solved by use of the method according to the present invention, which method is a volumetric measurement with real time temperature and pressure compensation.

Furthermore, according to yet another embodiment of the present invention, the volumetric measurement of the gas flow or gas sample is performed as a ruminant feed evaluation of the animal feed, which feed evaluation is directed to kinetic information, digestibility and/or metabolisable energy content.

Other areas of interest in relation to the present invention are in different fermentation applications. One such area of interest is in evaluating the metabolic activity of yeast or bacteria by determining the fermentative capacity/production or metabolic Intermediates via direct or indirect gas(es) emission or consumption.

Another such area of interest is in evaluating aerobic respiration analysis for dissolved organic matter or insoluble biomass. Such tests include but not limit to biological oxygen demand (BOD) analysis of water and wastewater, compostability analysis of insoluble organic matter and aerobic respiration measurement of soil sample for bioremediation. In above mentioned cases, oxygen gas or air need to be supplied continuously and oxygen gas or air consumption or up taking rate can be measured using the present invention with additional carbon dioxide adsorption step either *in-situ* or *ex-situ.* With such an arrangement, carbon dioxide gas which is produced via bacteria or cells metabolism can be adsorbed chemically using a suitable adsorption solution, such as alkaline solution (NaOH or KOH). Oxygen gas or air consumption or up taking rate is actually monitored using the present invention with all important kinetic information for data analysis. The advantages mentioned above apply in these cases when being compared to either conventional manual methods or complex chemical and gas composition analysis solutions used today.

Gas production measurements may be used for testing the yeast activity in various industries, e.g. the beer and bakery industry. The carbon dioxide evolution rate is used to measure the fermentative capacity. Specific rate of carbon dioxide production under anaerobic conditions with excess of sugar is defined as the fermentative capacity, and the best carbon dioxide producer is usually the best fermenter. At some stage during fermentation, carbon dioxide is produced maximally, and at this stage carbon dioxide evolution directly translates to substrate being consumed. Also the product accumulation will be at its maximum then. The present method employing a volumetric measurement has the advantage that it normalizes for the influence of varied ambient temperature and pressure, which is not the case of the manometric methods used today. Furthermore, the entire analysis in the method according to the present invention may be performed in constant pressure close to atmospheric pressure, which is also an advantage when being compared with manometric methods.

Furthermore, another application of interest with reference to the present invention is e.g. bioethanol fermentation. Also wine and ethanol fermentation for personal consumption and specific yeast stains development are areas where the method according to the present invention comes into play.

Moreover, also applications utilizing bacteria is of interest in relation to the present invention. For instance, lactic acid, which may be produced in a fermentative process using either bacteria or yeast, is a building block chemical with a wide range of applications (polymer, food and beverage, personal care, pharmaceutical), and as poly lactic acid it is used in the packaging and textile industries. Also here the production of carbon dioxide during fermentative production of lactic acid may be used as a measurement to determine the activity of producing microorganisms, study the kinetics and monitor such a process. Furthermore, the advantages mentioned above also apply in this case when being compared to manometric methods used today.

Furthermore, the Anammox process for nitrogen removal has gained increasing interest in wastewater treatment. Anammox bacteria activity analysis is needed to study the growth and enrichment of this specific bacteria group. The method according to the present invention may be used as a batch test platform to perform such bacteria activities test by monitoring production of nitrogen gas ($N_2$) without the need for off-line chemical analysis which is very labor and time consuming.

Moreover, the present invention also finds use in other applications. One example is for greenhouse gases emission control, which may be used in many different fields, such as waste/wastewater management, animal farming, agriculture et al. In connection to animal farming, the *in-vitro* analysis method according to the present invention may be used as a solution to develop and evaluate animal feed that can reduce the greenhouse gas emission from both animal breath and manure. Methane from cattle breath seems to give the biggest contribution on greenhouse gas emission and this has to be changed in order to meet the greenhouse gas emission reduction goal from the animal farming sector. In connection to animal waste handling, the method or system according to the present invention may be used as a batch platform for various batch fermentation tests following specific protocols to simulate the greenhouse gas emission from manure storage and waste handling.

It should further be said that the method according to the present invention may of course be utilized in any field of biogas, biomethane and landfill gases production for analyzing such processes based on the gas being produced.

The method according to the present invention provides a simple process for continuously monitoring gas production in many different applications, which methods does not create an overpressure. Since a volumetric measurement is performed according to the method and the data can be normalized for environmental conditions, the measurement is very robust and gives high quality results. As mentioned, the method according to the present invention is not very susceptible to experimental variations and errors as is the case of manometric methods.

Moreover, the present method may be used to analyze several gas components within the fields disclosed above. According to one specific embodiment, the measurement according to the method of the present invention is performed so as to distinguish between at least one gas component and the total gas. There is no indication that only methane and carbon dioxide will be produced in process discussed above. For instance, gases such as hydrogen or dihydrogen sulphur or nitrogen or oxygen may also be produced, and gas mixtures with more than two gas compounds from a list of possible gases including carbon dioxide ($CO_2$), methane ($CH_4$), hydrogen ($H_2$), nitrogen ($N_2$), oxygen ($O_2$) and dihydrogen sulphur ($H_2S$) etc may well be the produced mixtures being analyzed. Both total gas volume measurement and individual gas component analysis may be employed according to the present invention.

According to another aspect, the present invention is directed to a gas flow measurement device according to appended claim 5, having a gas inlet, a gas outlet and a gas compartment (or flow cell) with a definite and predefined inner geometric physical volume, the gas compartment having one gas accumulating end and one lifting end and having a pivoting element enabling the gas compartment to pivot upwards to release the gas contained therein and then downwards again to its initial position and operating according to the leverage effect, and said gas flow measurement device also comprising a closed wet space compartment (or chamber for the flow cell), where said gas compartment is arranged inside the closed wet space compartment, said gas flow measuring device being configured for performing a gas analysis measurement by a volumetric measurement of a gas flow by using the gas flow measurement device in batch fermentation assay including anaerobic and aerobic respiration analysis or in *in-vitro* analysis and wherein the gas flow measuring device comprises a closed wet space compartment comprising a flow cell chamber and a flow cell chamber cover, wherein the gas compartment is only connected to the outside via the gas inlet and the gas outlet, the gas compartment also having at least one sensor, for a volumetric measurement of the gas flow.

According to one specific embodiment of the present invention, the gas flow measuring device comprises a gas tube with a gas inlet port and a gas bubble outlet. According to yet another embodiment, the gas flow measuring device comprises a closed wet space compartment comprising a flow cell chamber and a flow cell chamber cover, which are attachable and detachable to each other, and also comprising a sealing ring sealing the flow cell chamber cover to the flow cell chamber. Alternatively, the flow cell chamber and flow cell chamber cover can also be welded together permanently without using any sealing ring.

Furthermore, according to yet another specific embodiment, the gas flow measurement device also comprises a pressure sensor and/or a temperature sensor. First of all, it may be mentioned that the gas volume measurement is heavily influenced by temperature and pressure during the analysis. It is therefore important to state the temperature and pressure condition when gas volume is presented. Moreover, off-gas from biological fermentation process is often moisture saturated wet gases. Moisture (water vapour) does contribute to gas volume and the level of contribution depends on temperature and pressure. Higher moisture content can be found at higher temperature and so on.

For wet gas volume measurements in different analysis conditions, such as biomethane potential test at lab environment and *in-vitro* digestibility for feed and food, it is therefore important to normalize gas volume to standard condition, i.e. 0°C, 1 atm and zero moisture, so different volume measurements can be compared at some condition despite various measuring conditions. Since environmental temperature and pressure may vary over time, the best way is to have on-line temperature and pressure sensors for real-time temperature and pressure compensation based on the ideal gas law equation. Since temperature is known and off-gas from fermentation can always be assumed as water saturated gases, it is also possible to calculate the volume contribution from moisture in order to calculate the volume of dry gas. This is the reason behind providing a gas volume measurement with temperature and pressure analysis, such as according to the present invention.

According to the present invention, the temperature and pressure sensors may be located close to a main electronic circuit board. Since it is very reasonable to assure the flow cell measurement is carried out in the same temperature as the environmental temperature, the temperature probe may be placed to monitor the variation of ambient temperature, and in case of a multi-arrangement, then all flow cells can share the same temperature measurement. Regarding the pressure, since it is intended to measure the gas volume without creating over- and under-pressure, it is also reasonable to assume that the gas volume measurement is carried out at ambient pressure. Therefore, according to the present invention the methid is performed by monitoring ambient temperature and pressure and use the values for real-time gas volume normalization.

It should also be mentioned that is also totally possible to put a pressure probe inside the gas compartment / flow cell chamber according to the present invention. In this case, any over and under pressure can also be monitored.

Furthermore, it may also be mentioned that that the design of the flow cell geometric shape may also be varied for having different flow cell volume resolutions.

Furthermore, and as hinted above, the present invention is also directed to a detection unit comprising several gas flow measurement devices according to the present invention, wherein all gas flow measurement devices each comprise one gas compartment contained in one closed wet space compartment and wherein all gas flow measurement devices are independently connected in series.

Furthermore, it should be noted that the gas flow measuring device according to the present invention also may function for vacuum or under pressured systems, i.e. gas(es) are continuously depleted, where the measuring device then gives a reading on the gas flow going from the gas flow measuring device and back to a connected vacuum held system. This is yet another advantage with the closed wet space system according to the present invention in comparison to open systems existing today.

Moreover, the present invention is also directed to the use of a gas flow measurement device, said gas flow measuring device comprising a gas inlet and at least one gas compartment with a definite and predefined inner geometric physical volume and active volume, the gas compartment having one gas accumulating end and one lifting end and having a pivoting element enabling the gas compartment to pivot upwards to release the gas contained therein and then downwards again to its initial position and operating according to the leverage effect, the gas compartment also having at least one sensor, for a volumetric measurement of the gas flow in batch fermentation and/or an *in-vitro* analysis platform.

As mentioned above, one important difference in relation to the flow measurement devices used in this field today is the use of a volumetric measurement according to the present invention instead of manometric. The fact that the system according to the present invention does not use either over- or under-pressures renders several advantages, such as high accuracy and precision and no need for over and under pressure build up to be detected by a pressure sensor in manometric principle. The system according to the present invention allows users to measure low gas volume and flow whenever there is a demand for accurate and precise measurements. The system according to the present invention provides an automatic platform which may be used for both research and industrial applications, e.g. in animal or human nutrition studies, wastewater treatment, composting, bioremediation, ethanol fermentation, biogas, biomethane, landfill gases and hydrogen production, greenhouse gas emissions and more, as discussed above.

The system according to the present invention reduces the labor demands as on-line real-time measurements of low gas flows produced from any gas generating process at laboratory scale may be generated.

The system has several use areas. According to one specific embodiment, it is used for an *in-vitro* analysis test. Therefore, according to one specific embodiment, the volumetric measurement of the gas flow in the gas flow measurement device is performed in an *in-vitro* analysis test. In this context it should be mentioned that the system may also be used as a batch fermentation test system, in all different possible areas, e.g. within the field of yeast or bacteria fermentation, also in the gut microbiology field.

According to one specific embodiment, the system or method according to the present invention is intended for evaluating the specific characteristics of animal feed/nutrition(s) or human food/nutrition(s) by determining the digestibility; or evaluating the metabolic activity of yeast or bacteria by determining the fermentative capacity/production or metabolic Intermediates via direct or indirect gas(es) emission. This is further discussed above. Moreover, according to yet another specific embodiment, the system is intended as an *in-vitro* digestibility analysis test for evaluating the specific characteristics of animal feed/nutrition(s) or human food/nutrition(s) by determining the digestibility. One specific embodiment is where the volumetric measurement of the gas flow or gas sample is used for ruminant feed evaluation of the animal feed, which feed evaluation is directed to kinetic information, digestibility and/or metabolisable energy content.

The gas flow measuring device is directed to volumetric measurement and should be able to detect very low flows.

According to one specific embodiment, the gas flow measuring device operates by liquid displacement and buoyancy.

According to the present invention, the at least one gas compartment is arranged in a closed compartment implying that it is only connected to the outside via a gas inlet and gas outlet. In this context a close compartment implies a flow cell compartment having e.g. a lid, thus the headspace gas inside the flow cell compartment is only connected to outside via gas inlet and outlet. This will allow to keep the headspace gas composition very close to the gas mix to be analyzed. The idea is to minimize the solubility effect of gas in the liquid solution which is also in small volume according to the current embodiment. With a close compartment, the equilibrium between gas phase in headspace of compartment and dissolved gas in the liquid can be more easily achieved. This may be important for measuring ultra low gas volumes and for gases that have high solubility in solution, like $CO_2$.

Also the actual flow measuring device may of course have specific features. Examples are such flow cells where a gas storing capacity of an inside of the gas compartment means is larger at the gas accumulating end than at the lifting end. Furthermore, the gas accumulating end may have a higher vertical position than the lifting end at an initial standby position. Moreover, the gas compartment means may have a triangular cross section, with sharp edges or rounded edges, the cross section being perpendicular to a longitudinal direction of the gas compartment means.

Furthermore, the at least one gas compartment may comprise a pressure sensor and a temperature sensor. This may be incorporated to enable to normalize the gas volume or flow to 0°C, 1 atm and possible zero moisture content if the gas is water saturated. According to the present invention, temperature and pressure real-time compensation for gas volume and flow normalization, may performed in different ways. Getting real-time temperature and pressure reading may be obtained by monitoring ambient temperature and pressure in real-time or temperature and pressure inside the gas flow measuring device, i.e. either in the closed compartment headspace or close to the gas bubble releasing spot.

There are different ways to analyze or estimate the gas composition. One way is to apply suitable on-line sensors. This can potentially be done for $CO_2$, $CH_4$, $(O_2)$, $H_2$. Another possibility is to absorb or adsorb other gas components and analysis the volume of the target gas component(s), such as by absorbing $CO_2$ and analyze $CH_4$ gas volume only.

Moreover, the gas flow measuring device may comprise several gas compartments. This provides a system with a modularized arrangement for simple production, maintenance and replacement.

Moreover, and as mentioned, the system according to the present invention may be utilized in many different industrial applications. One such is as an *in-vitro* test platform.

Detailed description of the drawings

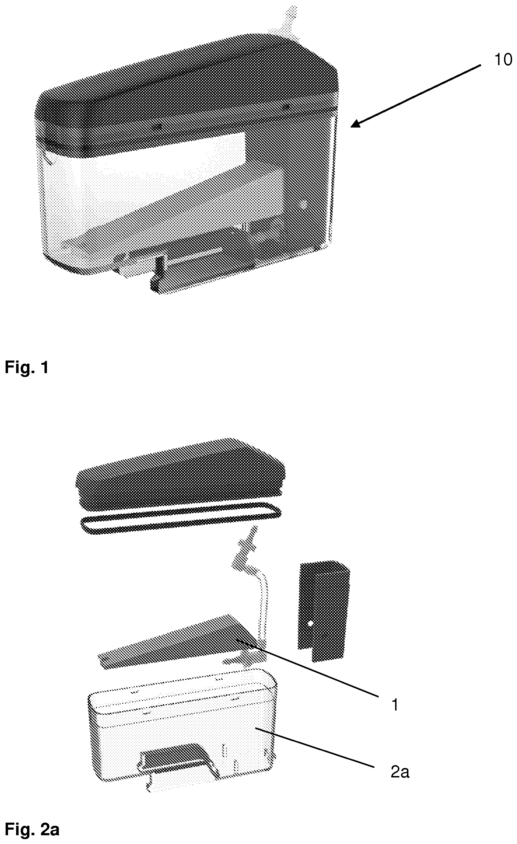

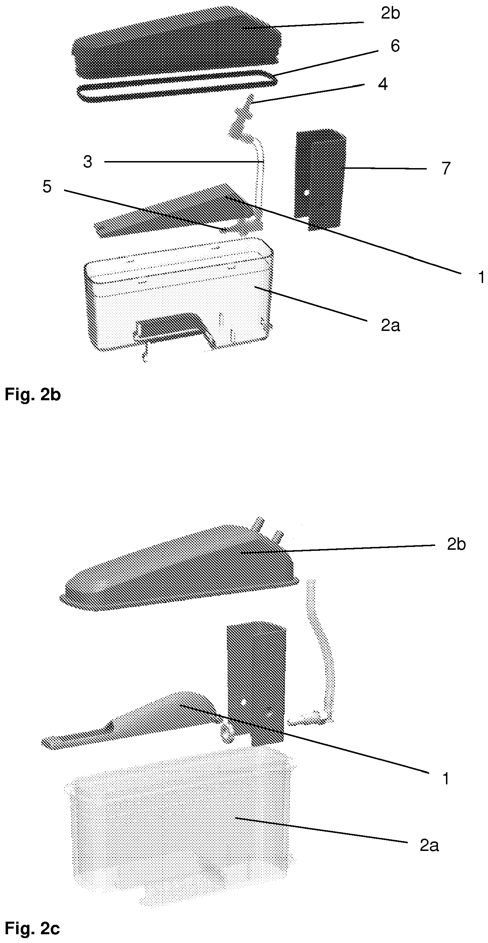

Fig. 1 shows a gas flow measuring device suitable for a system according to the present invention. Figs. 2a and b also show that gas flow measuring device, however in this case all parts are totally separated. As notable the gas flow measuring device in this case has a closed compartment. As may be seen, the gas flow measurement device 10 comprises a gas compartment 1 which is arranged in a closed wet space compartment 2. Furthermore, the actual gas compartment 1 has a predefined inner geometric physical volume and active volume. Moreover, the gas compartment 1 has one gas accumulating end and one lifting end and has a pivoting element enabling the gas compartment to pivot upwards to release the gas contained therein and then downwards again to its initial position.

Moreover, the device 10 comprises gas tube 3 with a gas inlet port 4 and a gas bubble outlet 5. Furthermore, the closed wet space compartment 2 comprises a flow cell chamber 2a and a flow cell chamber cover 2b, which are attachable and detachable to each other, and also a sealing ring 6.

Moreover, in fig. 2c there is shown yet another embodiment of the present invention. In this case sealing is enabled by direct welding so that the flow cell chamber 2a and flow cell chamber cover 2b are permanently welded together, instead of using a sealing ring.

In the figs. 2a-2c also the flow cell stand 7 is depicted.

Fig. 3 shows a schematic block diagram of one possible method according to the present invention, where the steps thereof are presented in the blocks. In this case the method presented may function as an *in-vitro* test platform for the evaluation of e.g. an animal feed.

Fig. 4-6 show diagrams of different trials when the method according to the present invention was evaluated. Fig. 4 demonstrates temperature and pressure variation over a 35-day period. The purpose of this plot is to demonstrate the importance of real-time temperature and pressure compensation for gas volume measurement.

Fig. 5a & 5b demonstrate the importance of gas volume normalization, i.e. an error will be introduced if one does not present gas volume at defined condition. Therefore it is improtant to state under what condition gas volume can be compared each other in different lab or literatures. The instrument used in this embodiment according to the present invention can perform real-time temperature and pressure compensation for gas volume measurement and all data is presented in standard condition (0°C 1atm and zero moisture content).

Furthermore, fig. 6 is an example of *in-vitro* digestability test for starch and grass using ruminant fluid. The digestability kinetic is clearly visiable.

Moreover, figs. 4-6 are further expained below.

Examples

A system according to the present invention was used to evaluate and prove the high accuracy and precise quantitative measurement possible to achieve with the volumetric gas measurement used. In this work, results from various biochemical methane potential tests were given, highlighting the importance of a correct adjustment of the quantitative gas measurements. It was shown that the varying ambient pressure and temperature can have a significant effect on the measured accumulated gas volume. Some preliminary result of an *in-vitro* feed digestibility test was also performed with the measuring device system according to the present invention, showing a clear correlation between the measured accumulated gas volume and the substrate concentrations used. In general, the variation between triplicates was minimal.

The variation of ambient pressure can significantly influence gas volume and flow measurement. To minimize the influence of ambient pressure difference and variation among different testing sites and labs, the gas volume is usually corrected to standard conditions using the ideal gas law. However, it should be considered that there are two common standard conditions which differ from each other on reference temperature (i.e. 0°C or 20°C).

In order to meet the measuring demand in high accuracy and precision, it is not just important that the pressure is measured as off line spot check, it should be measured continuously, at each measuring point in real time, to be sure a correct value is registered. The ambient pressure can vary from day to day which will give impact on both the dynamic profile and the accumulated volume.

As with the pressure, the temperature at measuring point will affect the volume of the gas and should be adjusted to standard conditions using the ideal gas law. The equation below shows how to adjust a gas volume to standard volume and pressure based on the ideal gas law.

$$V_{STP} = \frac{p_{STP}}{p_{gas}} * \frac{T_{gas}}{T_{STP}} V_{gas}$$

In the equation, $V_{STP}$ is the volume adapted to standard temperature and pressure, $p_{STP}$ is the standard pressure, $p_{gas}$ is pressure of the measured gas, $T_{gas}$ is the temperature of the measured gas, $T_{STP}$ is the standard temperature (which e.g. may be 0°C) and $V_{gas}$ is the measured volume.

Gas produced from anaerobic digestion and *in-vitro* digestibility tests is assumed to be saturated with water vapor and, in order to give accurate and correct quantitative gas measurements, the volume of water vapor should be removed. At the ranges where an anaerobic digestion test and *in-vitro* digestibility tests normally are performed (i.e. 0.9-1.1 bar and 10-40°C), the vapor pressure of water can satisfactory be approximated using the Antoine equation (see equation below). In this equation, $p_{vap}$ is the fraction of water vapor in the gas and $T_{gas}$ is the temperature of the gas in °C.

$$p_{vap} = 10^{8.1962 - \frac{1730.63}{233.426 + T_{gas}}}$$

Data was collected from a biochemical methane potential test carried out in a temperature controlled and well ventilated lab in Lund, Sweden. The recorded variation of pressure and temperature can be seen in Fig. 4. As can be seen, the pressure varies rather much even though the temperature can be remained in stable under a well-controlled lab environment.

When the three different factors (temperature, pressure and water vapor) are assumed to be constant, an error is introduced in the measurement. Fig. 5 shows the difference in the measured accumulated volume for the three different scenarios. The right hand side figure shows the variation in the relative error. As can be seen, the introduced error varies for the scenario where the pressure is assumed constant. The errors introduced by fixing the temperature or including water vapor are more constant in time.

An *in-vitro* digestibility test with rumen fluid has been performed, whereby different concentrations of starch and urea, and one concentration of a standard grass were used. The accumulated gas volume is monitored over time, and the pH is measured at the end of incubation, after circa 14 hours. The test was performed in triplicate. The average results are plotted in Fig. 6.

As can be seen, the standard deviation of the measured gas volume within the triplicates was in general very low, with exception for the triplicate with the highest concentrations of starch and urea (respectively 9g and 600 mg). This suggests that the accumulation of the produced fatty acids and the resulting low pH of 5.5 was limiting further digestion. This could also be the sign of feed overloading in the test vessels, as well as the reason for the higher standard deviation within the triplicates with high concentrations of starch and urea. A clear correlation can be seen between substrate concentration and gas production, whereby the grass results in a relatively low accumulated gas volume but a quick start of the digestion. This could be caused by easily digestible sugars that are present in the grass.

To further validate the instrument for *in-vitro* feed digestibility tests, a long term incubation of 96 hours was also performed. The method was compared to the standard VOS analysis, whereby the remaining organic matter amount, and thereby the organic matter digestibility *in vitro,* was determined after the incubation. The measuring device system according to the present invention resulted in a slightly higher remaining organic matter amount whereby the relative error compared to the VOS analysis was circa 3% (results not shown), except for the blank sample (only rumen fluid and buffer) where the relative error was higher. Overall, the results from the two methods are well correlated.

**Claims**

1. Method for performing a gas analysis measurement, said method comprising directing a gas flow or gas sample to a gas flow measurement device (10), said gas flow measuring device (10) comprising a gas inlet, and a gas compartment (1) with a definite and predefined inner geometric physical volume, the gas compartment (1) having one gas accumulating end and one lifting end and having a pivoting element enabling the gas compartment (1) to pivot upwards to release the gas contained therein and then downwards again to its initial position and operating according to the leverage effect,

**characterized in that** the gas flow measuring device comprises a gas outlet, the gas compartment (1) is arranged in a closed wet space compartment (2), implying that the gas compartment (1) is only connected to the outside via the gas inlet and the gas outlet, the gas compartment (1) also having at least one sensor, for a volumetric measurement of the gas flow, wherein the method is performed for evaluating a batch fermentation and/or in an in-vitro analysis platform, and wherein the method comprises that using the gas flow measurement device (10) is performed for evaluating the specific characteristics of animal feed/nutrition(s) or human food/nutrition(s) by determining the digestibility; or evaluating the activity of gut microbiota or yeast or bacteria by determining the fermentative capacity/production or bacterial metabolic activities.

2. Method according to claim 1, wherein the volumetric measurement of the gas flow or gas sample is performed as an *in-vitro* analysis test of the digestibility of the animal feed/nutrition(s) or the human food/nutrition(s) or activity of gut microbiota.

3. Method according to claim 1, wherein the volumetric measurement of the gas flow is performed in batch fermentation assays including anaerobic and aerobic respiration analysis.

4. Method according to claim 1 or 2, wherein the volumetric measurement of the gas flow or gas sample is performed as a ruminant feed evaluation of the animal feed, which feed evaluation is directed to kinetic information, digestibility and/or metabolisable energy content.

5. Gas flow measurement device (10) having a gas inlet, and a gas compartment (1) with a definite and predefined inner geometric physical volume, the gas compartment having one gas accumulating end and one lifting end and having a pivoting element enabling the gas compartment to pivot upwards to release the gas contained therein and then downwards again to its initial position and operating according to the leverage effect,
**characterized in that** the gas flow measurement device comprises a gas outlet and a closed wet space compartment (2), where said gas compartment (1) is arranged inside the closed wet space compartment (2), said gas flow measuring device (10) being configured for performing a gas analysis measurement by a volumetric measurement of a gas flow by using the gas flow measurement device (10) in batch fermentation assays including anaerobic and aerobic respiration analysis or in *in-vitro* analysis, and wherein the gas flow measuring device (10) comprises a closed wet space compartment (2) comprising a flow cell chamber (2a) and a flow cell chamber cover (2b), wherein the gas compartment (1) is only connected to the outside via the gas inlet and the gas outlet, the gas compartment (1) also having at least one sensor, for a volumetric measurement of the gas flow.

6. Gas flow measurement device (10) according to claim 5, wherein the gas flow measuring device (10) comprises a gas tube (3) with a gas inlet port (4) and a gas bubble outlet (5).

7. Gas flow measurement device (10) according to claim 5 or 6, wherein the flow cell chamber (2a) and the flow cell chamber cover (2b) are attachable and detachable to each other, and wherein the gas flow measuring device (10) also comprises a sealing ring (6) sealing the flow cell chamber cover (2b) to the flow cell chamber (2a).

8. Gas flow measurement device (10) according to claim 5 or 6, wherein the flow cell chamber (2a) and the flow cell chamber cover (2b) are sealed by direct welding.

9. Gas flow measurement device (10) according to claim any of claims 5-8, wherein the gas flow measurement device also comprises a pressure sensor and/or a temperature sensor.

10. Detection unit comprising several gas flow measurement devices (10) according to any of claims 5-9, wherein all gas flow measurement devices (10) are independently connected and placed in a serie.

## Patentansprüche

1. Verfahren zur Durchführung einer Gasanalysemessung, wobei das Verfahren Leiten eines Gasstroms oder einer Gasprobe zu einer Gasdurchflussmessvorrichtung (10) umfasst, wobei die Gasdurchflussmessvorrichtung (10) einen Gaseinlass und einen Gasraum (1) mit einem eindeutigen und vordefinierten geometrischen physikalischen Innenvolumen umfasst, wobei der Gasraum (1) ein gasakkumulierendes Ende und ein Hebeende aufweist und ein Schwenkelement aufweist, das ermöglicht, dass der Gasraum (1) nach oben schwenkt, um das darin enthaltene Gas freizugeben, und dann wieder nach unten in seine Ausgangsstellung, und nach der Hebelwirkung arbeitet,
**dadurch gekennzeichnet, dass** die Gasdurchflussmessvorrichtung einen Gasauslass umfasst, der Gasraum (1) in einer geschlossenen Nassraumkammer (2) angeordnet ist, was bedeutet, dass der Gasraum (1) nur über den Gaseinlass und den Gasauslass mit dem Außenraum verbunden ist, wobei der Gasraum (1) ferner wenigstens einen Sensor für eine volumetrische Messung des Gasstroms aufweist, wobei das Verfahren zur Bewertung einer Batchfermentation und/oder in einer *In-vitro*-Analyseplattform durchgeführt wird und wobei das Verfahren umfasst, dass die Verwendung der Gasdurchflussmessvorrichtung (10) zur Bewertung der spezifischen Merkmale von Tierfutter/-nahrung(en) oder menschlichen Lebensmitteln/Nahrung(en) durch Bestimmung der Verdaubarkeit; oder Bewertung der Aktivität von Darm-Mikrobiota oder -Hefe oder -Bakterien durch Bestimmung der Fermentationskapazität/-produktion oder von bakteriellen Stoffwechselaktivitäten durchgeführt wird.

2. Verfahren gemäß Anspruch 1, wobei die volumetrische Messung des Gasstroms oder der Gasprobe als *In-vitro*-Analyseprüfung der Verdaubarkeit des/der Tierfutters/- nahrung(en) oder der menschlichen Lebensmittel/Nahrung(en) oder der Aktivität von Darm-Mikrobiota durchgeführt wird.

3. Verfahren gemäß Anspruch 1, wobei die volumetrische Messung des Gasstroms in Batch-Fermentationsassays, einschließlich anaerober und aerober Respirationsanalyse, durchgeführt wird.

4. Verfahren gemäß Anspruch 1 oder 2, wobei die volumetrische Messung des Gasstroms oder der Gasprobe als Bewertung von Wiederkäuerfutter des Tierfutters durchgeführt wird, wobei die Futterbewertung auf kinetische Information, Verdaubarkeit und/oder metabolisierbaren Energiegehalt gerichtet ist.

5. Gasdurchflussmessvorrichtung (10), die einen Gaseinlass und einen Gasraum (1) mit einem eindeutigen und vordefinierten geometrischen physikalischen Innenvolumen aufweist, wobei der Gasraum ein gasakkumulierendes Ende und ein Hebeende aufweist und ein Schwenkelement aufweist, das ermöglicht, dass der Gasraum nach oben schwenkt, um das darin enthaltene Gas freizugeben, und dann wieder nach unten in seine Ausgangsstellung, und nach der Hebelwirkung arbeitet, **dadurch gekennzeichnet, dass** die Gasdurchflussmessvorrichtung einen Gasauslass und eine geschlossene Nassraumkammer (2) umfasst, wobei der Gasraum (1) innerhalb der geschlossenen Nassraumkammer (2) angeordnet ist, wobei die Gasdurchflussmessvorrichtung (10) dafür gestaltet ist, eine Gasanalysemessung durch eine volumetrische Messung eines Glasflusses unter Verwendung der Gasdurchflussmessvorrichtung (10) in Batch-Fermentationsassays, einschließlich anaerober und aerober Respirationsanalyse oder In-vitro-Analyse, durchzuführen, und wobei die Gasdurchflussmessvorrichtung (10) eine geschlossene Nassraumkammer (2) umfasst, die eine Durchflusszellenkammer (2a) und eine Durchflusszellenkammer-Abdeckung (2b) umfasst, wobei der Gasraum (1) nur über den Gaseinlass und den Gasauslass mit dem Außenraum verbunden ist, wobei der Gasraum (1) ferner wenigstens einen Sensor für eine volumetrische Messung des Gasstroms aufweist.

6. Gasdurchflussmessvorrichtung (10) gemäß Anspruch 5, wobei die Gasdurchflussmessvorrichtung (10) ein Gasrohr (3) mit einer Gaseinlassöffnung (4) und einem Gasblasenauslass (5) umfasst.

7. Gasdurchflussmessvorrichtung (10) gemäß Anspruch 5 oder 6, wobei die Durchflusszellenkammer (2a) und die Durchflusszellenkammer-Abdeckung (2b) aneinander befestigbar und voneinander ablösbar sind und wobei die Gasdurchflussmessvorrichtung (10) ferner einen Dichtungsring (6) umfasst, der die Durchflusszellenkammer-Abdeckung (2b) gegenüber der Durchflusszellenkammer (2a) abdichtet.

8. Gasdurchflussmessvorrichtung (10) gemäß Anspruch 5 oder 6, wobei die Durchflusszellenkammer (2a) und die Durchflusszellenkammer-Abdeckung (2b) durch direktes Schweißen versiegelt sind.

9. Gasdurchflussmessvorrichtung (10) gemäß einem der Ansprüche 5-8, wobei die Gasdurchflussmessvorrichtung ferner einen Drucksensor und/oder einen Temperatursensor umfasst.

10. Nachweiseinheit, umfassend mehrere Gasdurchflussmessvorrichtungen (10) gemäß einem der Ansprüche 5-9,
wobei alle Gasdurchflussmessvorrichtungen (10) unabhängig angeschlossen und in einer Reihe angeordnet sind.


**Revendications**

1. Procédé pour réaliser une mesure d'analyse de gaz, ledit procédé comprenant l'orientation d'un écoulement de gaz ou échantillon de gaz vers un dispositif de mesure d'écoulement de gaz (10), ledit dispositif de mesure d'écoulement de gaz (10) comprenant un entrée de gaz, et un compartiment de gaz (1) avec un volume physique géométrique intérieur défini et prédéfini, le compartiment de gaz (1) ayant une extrémité d'accumulation de gaz et une extrémité de levage et ayant un élément pivotant permettant au compartiment de gaz (1) de pivoter vers le haut pour libérer le gaz contenu dans celui-ci et puis vers le bas à nouveau jusqu'à sa position initiale et fonctionnant selon l'effet de levier, **caractérisé en ce que** le dispositif de mesure d'écoulement de gaz comprend une sortie de gaz, le compartiment de gaz (1) est agencé dans un compartiment d'espace humide fermé (2), ce qui implique que le compartiment de gaz (1) est seulement raccordé à l'extérieur par l'intermédiaire de l'entrée de gaz et de la sortie de gaz, le compartiment de gaz (1) ayant également au moins un capteur, pour une mesure volumétrique de l'écoulement de gaz, dans lequel le procédé est réalisé pour évaluer une fermentation à écoulement discontinu et/ou dans une plate-forme d'analyse in vitro, et dans lequel le procédé comprend le fait de l'utilisation du dispositif de mesure d'écoulement de gaz (10) est réalisée pour évaluer les caractéristiques spécifiques de nourriture/nutrition(s) d'animal ou d'aliment/nutrition(s) d'humain en déterminant la digestibilité ; ou évaluer l'activité de microbiote intestinal ou de levure ou de bactérie en déterminant la capacité/production fermentative ou des activités métaboliques bactériennes.

2. Procédé selon la revendication 1, dans lequel la mesure volumétrique de l'écoulement de gaz ou de l'échantillon de gaz est réalisée sous forme d'essai d'analyse in vitro de la digestibilité de la (des) nourriture/nutrition(s) d'animal ou de (des) l'aliment/nutrition(s) d'humain ou de l'activité de microbiote intestinal.

3. Procédé selon la revendication 1, dans lequel la mesure volumétrique de l'écoulement de gaz est réalisé dans des analyses de fermentation à écoulement discontinu incluant une analyse de respiration anaérobie et aérobie.

4. Procédé selon la revendication 1 ou 2, dans lequel la mesure volumétrique de l'écoulement de gaz ou de l'échantillon de gaz est réalisée sous forme d'évaluation de nourriture de ruminant de la nourriture d'animal, laquelle évaluation de nourriture cible des informations cinétiques, la digestibilité et/ou la teneur en énergie métabolisable.

5. Dispositif de mesure d'écoulement de gaz (10) ayant une entrée de gaz, et un compartiment de gaz (1) avec un volume physique géométrique intérieur défini et prédéfini, le compartiment de gaz ayant une extrémité d'accumulation de gaz et une extrémité de levage et ayant un élément pivotant permettant au compartiment de gaz de pivoter vers le haut pour libérer le gaz contenu dans celui-ci et puis vers le bas à nouveau jusqu'à sa position initiale et fonctionnant selon l'effet de levier,
**caractérisé en ce que** le dispositif de mesure d'écoulement de gaz comprend une sortie de gaz et un compartiment d'espace humide fermé (2), où ledit compartiment de gaz (1) est agencé à l'intérieur du compartiment d'espace humide fermé (2), ledit dispositif de mesure d'écoulement de gaz (10) étant configuré pour réaliser une mesure d'analyse de gaz par le biais d'une mesure volumétrique d'un écoulement de gaz en utilisant le dispositif de mesure d'écoulement de gaz (10) dans des analyses de fermentation à écoulement discontinu incluant une analyse de respiration anaérobie et aérobie ou dans une analyse in vitro, et dans lequel le dispositif de mesure d'écoulement de gaz (10) comprend un compartiment d'espace humide fermé (2) comprenant une chambre cellule d'écoulement (2a) et un couvercle de chambre cellule d'écoulement (2b), dans lequel le compartiment de gaz (1) est seulement raccordé à l'extérieur par l'intermédiaire de l'entrée de gaz et de la sortie de gaz, le compartiment de gaz (1) ayant également au moins un capteur, pour une mesure volumétrique de l'écoulement de gaz.

6. Dispositif de mesure d'écoulement de gaz (10) selon la revendication 5, dans lequel le dispositif de mesure d'écoulement de gaz (10) comprend un tube de gaz (3) avec un orifice d'entrée de gaz (4) et une sortie de bulles de gaz (5).

7. Dispositif de mesure d'écoulement de gaz (10) selon la revendication 5 ou 6, dans lequel la chambre cellule d'écoulement (2a) et le couvercle de chambre cellule d'écoulement (2b) sont attachables et détachables l'un à l'autre et dans lequel le dispositif de mesure d'écoulement de gaz (10) comprend également une bague d'étanchéité (6) étanchéifiant le couvercle de chambre cellule d'écoulement (2b) sur la chambre cellule d'écoulement (2a).

8. Dispositif de mesure d'écoulement de gaz (10) selon la revendication 5 ou 6, dans lequel la chambre cellule d'écoulement (2a) et le couvercle de chambre cellule d'écoulement (2b) sont étanchéifiés par soudage direct.

9. Dispositif de mesure d'écoulement de gaz (10) selon l'une quelconque des revendications 5 à 8, dans lequel le dispositif de mesure d'écoulement de gaz comprend également un capteur de pression et/ou un capteur de température.

10. Unité de détection comprenant plusieurs dispositifs de mesure d'écoulement de gaz (10) selon l'une quelconque des revendications 5 à 9,
dans laquelle tous les dispositifs de mesure d'écoulement de gaz (10) sont indépendamment raccordés et placés en une série.

10

**Fig. 1**

1

2a

**Fig. 2a**

2b

6

4

3

5

7

1

2a

**Fig. 2b**

2b

1

2a

**Fig. 2c**

| A process or digest system where microbials or enzymes will interact with substrates or feed | → | Biological or enzymatic reactions result in fermentation product(s) or intermediate metabolite(s) together with direct or indirect gas(es) production | → | Gasvolume or/and gas composition analysis | → | Microchip or computer based DAQ (continuous, real-time) |

**Fig. 3**

**Fig. 4**

**Fig. 5a and 5b**

**Fig. 6**

**EP 3 458 816 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012064616 A
- WO 2010120229 A
- WO 2010120230 A
- US 4064750 A
- GB 2531331 A